# EUROPÄISCHE PATENTSCHRIFT

(11) **EP 2 121 549 B1**
(45) Veröffentlichungstag und Bekanntmachung des Hinweises auf die Patenterteilung: **07.06.2017**
(21) Anmeldenummer: 07726908.2
(22) Anmeldetag: 14.03.2007
(51) Int. Cl.: C07C 29/42, C07C 29/17

(54) **VERFAHREN ZUR HERSTELLUNG VON 1,4-BUTANDIOL**
METHOD FOR THE PRODUCTION OF 1,4-BUTANEDIOL
PROCÉDÉ DE PREPARATION DE 1,4-BUTANEDIOL

(30) Priorität: 15.02.2007 EP 07102460
(43) Veröffentlichungstag der Anmeldung: 25.11.2009
(73) Patentinhaber: BASF SE, 67056 Ludwigshafen am Rhein (DE)
(72) Erfinder: PINKOS, Rolf, 67098 Bad Dürkheim (DE); LORENZ, Rudolf Erich, 67245 Lambsheim (DE); BESTE, York Alexander, 67346 Speyer (DE)
(74) Vertreter: BASF IP Association
(86) Internationale Anmeldenummer: PCT/EP2007/052417
(87) Internationale Veröffentlichungsnummer: WO 2008/098620

(56) Entgegenhaltungen:
- DE-A1- 2 357 751
- GB-A- 1 217 775

## Beschreibung

Die Erfindung betrifft ein Verfahren zur Herstellung von 1,4-Butandiol aus Acetylen und Formaldehyd über die Zwischenstufe 1,4-Butindiol.

Die Synthese von 1,4-Butindiol aus Acetylen und Formaldehyd wird industriell mehrfach betrieben und ist beispielsweise bei K. Weissermel, H.-J. Arpe, Industrielle organische Chemie, 5. Auflage, 1998, Wiley-VCH, Seiten 110 und 111) beschrieben worden. Neben Kupfer können die üblicherweise verwendeten Katalysatoren gegebenenfalls Wismut sowie Silikate (im Folgenden SiO₂ genannt) oder Aluminiumoxid enthalten. Während der Synthese von 1,4-Butindiol kommt es als Nebenreaktion zur Bildung von oligomerer beziehungsweise polymerer Substanzen (im folgenden Cuprene genannt). Diese Cuprene gelangen üblicherweise zusammen mit löslichen und unlöslichen Bestandteilen des verwendeten Katalysators in die Hydrierstufe, bei der zunächst 1,4-Butendiol gebildet wird, das sich in einem weiteren Hydrierschritt zum gegenüber 1,4-Butendiol bedeutsameren Zwischenprodukt 1,4-Butandiol hydrieren lässt.

Die Hydrierung von 1,4-Butindiol zu 1,4-Butandiol wird seit Jahrzehnten betrieben (GB 1 217 775 A) und ist vielfältig beschrieben. So ist aus der US-A 5 068 468 die Hydrierung von 1,4-Butandiol an festen geträgerten Nickel-Kupfer-Katalysatoren bekannt, während US-A 4 153 578 ein zweistufiges Verfahren zur Hydrierung von 1,4-Butindiol an suspendierten Raney-Nickel-Molybdän-Katalysatoren bei einem Druck von 21 bar beschreibt. Die DD-A 272 644 offenbart die Suspensionshydrierung von wässrigem Butindiol an Nickel-SiO₂-Katalysatoren und aus der EP-B 0 319 208, der DE-A 19 41 633 und der DE-A 20 40 501 sind allgemeine, unter anderem auf 1,4-Butindiol anwendbare, Hydrierverfahren bekannt.

Cuprene und Katalysatorbestandteile aus der Butindiolsynthese stören die Hydrierung von 1,4-Butindiol zu 1,4-Butendiol oder 1,4-Butandiol und verschlechtern das Hydrierergebnis deutlich. So lagern sich Cuprene auf dem Katalysator und den Poren ab und behindern den Kontakt von Butindiol mit der Katalysatoroberfläche mit der Folge, dass die Reaktion langsamer wird und der Druckverlust über den Reaktor ansteigt. Katalysatorkomponenten wie Kupfer, Wismut und/oder SiO₂ scheiden sich ebenfalls auf dem Katalysator ab und verändern zusammen mit den Cuprenen die Katalysatoraktivität und Selektivität.

Diese nachteiligen Effekte können neben einem Differenzdruckaufbau auch an der Bildung des Nebenprodukts Butanol und weiteren, die Reinheit des Butandiols störenden Komponenten, verfolgt werden, da deren Bildung durch die vorstehend genannten Katalysatorgifte beschleunigt wird.

Die einfache Reinigung von 1,4-Butindiol, z. B. durch Filtration ist kaum möglich, da insbesondere die Cuprene und SiO₂ zum Teil kolloidal beziehungsweise feinst verteilt vorliegen und somit gängige Filter schnell verstopfen, so dass die Filter entweder ständig ausgetauscht oder aufwändig rückgespült werden müssen.

Aufgabe der Erfindung ist es, ein großtechnisches Verfahren zur Verfügung zu stellen, mit dem, insbesondere bei Anlagen mit großen Kapazitäten, möglichst einfach und wirtschaftlich 1,4-Butandiol produziert werden kann, ohne dass die verwendeten Katalysatoren schnell an Aktivität einbüßen und regeneriert oder ausgetauscht werden müssen. Zudem soll 1,4-Butandiol zuverlässig in der vom Markt geforderter Reinheit hergestellt werden können. Im Allgemeinen ist dabei ein 1,4-Butandiol-Gehalt von mindestens 99,5 % gefordert, wobei daneben die Gehalte an 2-Methyl-1,4-butandiol max. 0,4 % und an 2-(4-Hydroxybutoxy)-tetrahydrofuran, im Folgenden Acetal genannt, max. 0,15 % betragen können.

Überraschenderweise wurde nun gefunden, dass diese Aufgabe durch ein integriertes Verfahren zur kontinuierlichen Herstellung von 1,4-Butandiol, das folgende Stufen umfasst,
(I) Umsetzung von Formaldehyd mit Acetylen in Gegenwart eines kupferhaltigen Katalysators bei einem pH-Wert von 5 bis 8 und einem Molverhältnis von Formaldehyd zu Acetylen von höchstens 2:1,
(II) Zwischenpufferung des erhaltenen Butindiol-haltigen wässrigen Gemischs für 0,1 bis 100h,
(III) Hydrierung des nach der Zwischenpufferung erhaltenen Gemischs und
(IV) Destillation des in Stufe III erhaltenen Hydrierprodukts zur Gewinnung von 1,4-Butandiol,
wobei der Reaktionsaustrag aus Stufe (I) direkt in die Zwischenpufferung (II) gefahren wird und nach der Zwischenpufferung (II) in mindestens einer Destillationsstufe (IIa) Acetylen, Formaldehyd, Wasser und Nebenprodukte abgetrennt werden und wobei unter Zwischenpufferung eine von dem Reaktor der Stufe (I) und den Hydrierreaktoren der Stufe (III) räumlich getrennter Bereich, der ein beliebiger Behälter, ein Tank, ein Rührapparat oder eine Rohrleitung sein kann, verstanden, in dem der Reaktionsaustrag für die erfindungsgemäße Verweilzeit verbleibt, gelöst wird.

Das für Stufe (I) des erfindungsgemäßen Verfahrens zu verwendende Acetylen kann beispielsweise durch Hydrolyse von Calciumacetylid (Carbid), partieller Verbrennung von Kohlenwasserstoffen wie Methan oder aus Crackern stammen. Es wird erfindungsgemäß bei einem Druck (absolut) von 0,5 bis 50 bar, bevorzugt bei 0,8 bis 25 bar, besonders bevorzugt bei 0,9 bis 10 bar mit Formaldehyd umgesetzt. Acetylen hat vorzugsweise eine Reinheit von > 99 %.

Formaldehyd wird in Stufe (I) des erfindungsgemäßen Verfahrens als wässrige Lösung eingesetzt und stammt im Allgemeinen aus der (partiellen) Oxidation vom Methanol.

Der Formaldehydgehalt in Wasser kann beispielsweise zwischen 10 und 80 Gew.-% liegen, bevorzugt liegt der Gehalt zwischen 25 und 65 Gew.-%, besonders bevorzugt liegt er zwischen 30 und 60 Gew.-%. Die Reinheit des Formaldehyds (wasserfrei gerechnet) sollte über 95 % liegen. Üblicherweise besteht der Rest zu 100 % vorwiegend aus Methanol, es ist jedoch vorteilhaft, wenn dessen Gehalt möglichst klein ist, d.h. die Reinheit des Formaldehyds deutlich über 95 % liegt. Weitere Nebenbestandteile wie z. B. Ameisensäure liegen vorteilhaft im Bereich von < 0,1 %.

Erfindungsgemäß wurde erkannt, dass es zur möglichst effizienten Umsetzung von Acetylen mit Formaldehyd bevorzugt ist, dass über die gesamte Reaktionszone für Stufe (I) immer freies Acetylen vorliegt, d.h. auch gegen Ende der Reaktionszone (Reaktorausgang). Unter freiem Acetylen wird in Rahmen dieser Erfindung Acetylen verstanden, das in gelöster Form oder gasförmig im Reaktor für die Umsetzung von Acetylen mit Formaldehyd vorhanden ist. Dies bedeutet, dass das Molverhältnis von Formaldehyd : Acetylen in der Reaktion bevorzugt höchstens 2 : 1 beträgt. Durch das Vorliegen freien Acetylens wird überraschenderweise die vorzeitige Alterung bzw. Zerstörung des Katalysators verhindert. Es ist deshalb bevorzugt, entweder zu Beginn der Reaktion bereits ein Molverhältnis Acetylen zu Formaldehyd von mindestens 0,5 zu 1 einzusetzen oder während der Reaktion an verschiedenen Stellen der Reaktionszone noch zusätzlich Acetylen einzudosieren. Besonders bevorzugt beträgt das Molverhältnis von Acetylen zu Formaldehyd in der oder den Reaktionszonen 0,501 zu 1 bis 0,55 zu 1.

In Stufe (I) wird die Umsetzung von Acetylen mit Formaldehyd üblicherweise bei Temperaturen von 30 bis 130°C durchgeführt. Bevorzugt sind 50 bis 100°C, besonders bevorzugt 65 bis 90°C.

Es werden Verweilzeiten von 0,5 bis 200 h, bevorzugt 1 bis 100 h, besonders bevorzugt 5 bis 50 h, eingestellt.

Die für die Umsetzung von Acetylen mit Formaldehyd verwendeten Katalysatoren sind an sich bekannt und geeignete Katalysatoren sind beispielsweise in DE-A 22 09 520, DE-A 22 09 521, DE-A 23 57 752, DE-A 23 57 751, DE-A 26 02 418, DE-A 197 53 458 beschrieben.

Bevorzugt werden in Stufe (I) des erfindungsgemäßen Verfahrens Katalysatoren verwendet, die Kupfer enthalten, noch weitere Komponenten wie Wismut aufweisen. Besonders bevorzugt werden, die aus DE-A 26 02 418 bekannten Wismut und Kupfer auf einem Siliciumdioxid-Träger aufweisenden Katalysatoren eingesetzt.

Die Umsetzung von Acetylen und Formaldehyd kann in einem oder mehreren parallelen oder hintereinander geschalteten Reaktoren erfolgen. Die dabei verwendeten Katalysatoren können dabei in Suspension, in einem Fließbett und/oder einem Festbett eingesetzt werden, wobei bei Verwendung mehrerer Reaktoren auch Ausführungsformen mit verschiedenen Fahrweisen in den einzelnen Reaktoren möglich sind.

Die Umsetzung von Formaldehyd und Acetylen (Stufe (I)) über ein Festbett kann in Riesel- als auch Sumpffahrweise betrieben werden. Es ist in diesem Fall bevorzugt, mindestens zwei aufeinanderfolgende Reaktoren zu benutzen, da dies die Reaktionsführung erleichtert und höhere Umsätze gewährleistet. Jeder Reaktor kann dabei für sich oder alle Reaktoren gemeinsam über einen externen Gas- und/oder Flüssigkeitsumlauf, beispielsweise ein Verhältnis Zulauf zu Flüssigumlauf von 1 zu 20, verfügen. Damit kann beispielsweise die Temperaturführung vereinfacht und/oder für eine möglichst hohe Acetylenkonzentration gesorgt werden. Werden mehrere Reaktoren verwendet, so können diese bei unterschiedlichen Temperatur- und Druckniveaus betrieben werden. Ferner kann damit, sofern ein oder mehrere Reaktoren in Sumpffahrweise betrieben werden, dafür gesorgt werden, dass sich die Katalysatorfüllung etwas anhebt, was einerseits zur Verminderung des auf dem Katalysator lastenden Gewichts führt, andererseits bessere Durchmischung gewährleistet.

Während der Umsetzung von Acetylen mit Formaldehyd kann durch eine geeignete Einstellung des pH-Werts eine Schädigung der Katalysatoren verhindert werden. Der pH-Wert für Stufe (I) beträgt 4 bis 10, bevorzugt 5 bis 8, besonders bevorzugt 6 bis 8., Die Einstellung des pH-Wertes kann durch die Zugabe von organischen oder anorganischen Säuren oder Basen oder Stoffen, die in Wasser sauer oder basisch reagieren, erfolgen. Die Zugabe kann beispielsweise in den Feedstrom und/oder den Kreislaufstrom erfolgen. Werden mehrere Reaktoren verwendet, so kann jeder Reaktor für sich pH-geregelt werden. Saure oder sauer wirkende Zusatzstoffe sind beispielsweise Schwefelsäure, Phosphorsäure, Salzsäure, Ameisensäure, Essigsäure, Ester wie Ethylformiat oder gamma-Butyrolacton. Im Regelfall ist die Dosierung von Basen oder basisch wirkenden Stoffen bedeutsamer, da sich durch Nebenreaktionen des Formaldehyds sauer wirkende Stoffe wie z.B. Ameisensäure bildet. Beispiele hierzu sind Hydroxide, Carbonate, Hydrogencarbonate, Formiate, Acetate, Oxalate der Alkali- und Erdalkalimetalle wie z.B. Natriumhydroxid, Kaliumhydroxid, Natriumcarbonat, Natriumhydrogencarbonat, Kaliumcarbonat oder Kaliumhydrogencarbonat. Bevorzugt werden diese Stoffe als wässrige Lösungen eingebracht. Es sind auch Mischungen der basischen oder der sauren Stoffe möglich. Bevorzugt wird wässrige Natron- oder Kalilauge verwendet.

Durch die pH-Regulierung werden zwar die Katalysatoren für Stufe (I) geschont, jedoch werden in das Reaktionssystem Salze eingebracht, die üblicherweise im Reaktionsaustrag der Umsetzung von Acetylen mit Formaldehyd verbleiben, da eine Abtrennung, z.B. durch Destillation oder Ionentausch sehr aufwändig ist und eine großtechnische Anwendung des Verfahrens wirtschaftlich weniger attraktiv machen würde. Der Reaktionsaustrag der Stufe (I) des erfindungsgemäßen Verfahrens enthält neben 10 bis 90 Gew.-% 1,4-Butindiol, den genannten Salzen, Wasser, unumgesetztem Formaldehyd, unumgesetztem Acetylen, kleineren Mengen Zwischenprodukte wie Propinol, Produkte aus der Umsetzung von Acetylen und Formaldehyd, die zum Teil einen höhermolekularen Charakter haben. So sind dies beispielsweise Formaldehydacetale auf Basis Methanol, Propinol und 1,4-Butindiol sowie oligomere und polymere Komponenten die z.T. gelöst, als Kolloide vorhanden oder mit dem bloßen Auge als Feststoffe erkennbar sind wie beispielsweise Cuprene (Chemie und Technik der Acetylen-Druck-Reaktionen, Walter Reppe, Weinheim, Verlag Chemie, 1951). Ferner können noch Katalysatorbestandteile in gelöster, kolloidaler und/oder fester Form enthalten sein. Je nach Katalysator sind dies beispielsweise Komponenten die Kupfer, Wismut oder Katalysatorträgermaterialien wie Aluminiumoxidhydrate, Silikate, Titanate und dergleichen enthalten.

Erfindungsgemäß wurde nun erkannt, dass es vorteilhaft ist, den Reaktionsaustrag der Stufe (I) in einen Zwischenpuffer einzuleiten, in dem die mittlere Verweilzeit möglichst hoch ist, technisch relevante Verweilzeiten liegen bei 0,1 bis 100 h, bevorzugt sind 0,5 bis 50 h, besonders bevorzugt bei 1 bis 30 h. Unter Zwischenpuffer wird in dieser Anmeldung ein von dem Reaktor der Stufe (I) und den Hydrierreaktoren der Stufe (III) räumlich getrennter Bereich, der ein beliebiger Behälter, ein Tank, ein Rührapparat oder eine Rohrleitung sein kann, verstanden, in dem der Reaktionsaustrag für die erfindungsgemäße Verweilzeit verbleibt. Bevorzugt wird ein Tank oder Behälter verwendet, der über eine Umpumpung oder Rührung verfügen kann. Vorteilhaft liegt die Entnahmestelle für den Zulauf in die Stufe (III) oberhalb der tiefsten Stelle des Tanks oder Behälters. Dies bewirkt, dass sich Feststoffe absetzen können und dadurch nicht in die Hydrierung gelangen.

Die Zwischenpufferung gemäß Stufe (II) des erfindungsgemäßen Verfahrens des 1,4-Butindiol enthaltenden, wässrigen Produktstroms der einen 1,4-Butindiol-Gehalt von beispielsweise 10 bis 90 Gewichts-%, bevorzugt von 30 bis 70 % und besonders bevorzugt von 40 bis 60 % aufweist, wird üblicherweise bei Temperaturen von 20 bis 100°C, bevorzugt bei 40 und 90°C, besonders bevorzugt sind 50 bis 80°C. Vorteilhafterweise wird die Zwischenpufferung unter Inertgasatmosphäre vollzogen. Als Inertgase können z. B. Stickstoff, Kohlendioxid oder die Edelgase dienen, bevorzugt wird Stickstoff verwendet. Der Druck ist für die Zwischenpufferung an sich unkritisch, bevorzugt wird die Zwischenpufferung jedoch bei 0,8 bis 20 bar durchgeführt. Der bevorzugte Temperaturbereich liegt zwischen 40 und 90°C, besonders bevorzugt sind 50 bis 80°C.

Es ist jedoch auch möglich, den Reaktionsaustrag aus Stufe (I) direkt in den Zwischenpuffer zu fahren und nach der Zwischenpufferung (Stufe II) und vor der Hydrierung zu 1.4-Butandiol (Stufe III). überschüssiges Acetylen bei 0 bis 100 °C und 0,1 bis 1 bar (absolut) zu entfernen. Das abgetrennte Acetylen kann in Stufe (I) zurückgeführt werden. Weiterhin kann in einer weiteren Ausführungsform zusätzlich überschüssiger Formaldehyd zusammen mit Wasser und/oder Zwischenprodukten wie Propinol destillativ beispielsweise bei 80 - 100°C und 0,1 - 1 bar (absolut) abgetrennt werden und in Stufe (I) rückgeführt werden. Nach Destillationsstufe (IIa) kann ein weiterer Zwischenpuffer unter den vorstehend für Stufe (II) genannten Bedingungen verwendet werden.

Überraschenderweise wurde gefunden, dass durch die Zwischenpufferung des Butindiol enthaltenden, gegebenenfalls vorher destillierten, wässrigen Produktstroms, die nachfolgende Hydrierung zum 1,4-Butandiol deutlich wirtschaftlicher wird, da einerseits die Katalysatorlebensdauer (Katalysatorstandzeit) und andererseits die Ausbeute an 1,4-Butandiol sowie dessen Reinheit verbessert wird.

Anschließend wird das nach der Zwischenpufferung (II) erhaltene Gemisch katalytisch hydriert (Stufe III).

Für die Hydrierung von 1,4-Butindiol sind solche Katalysatoren geeignet, die C-C-Dreifach- und -Doppelbindungen zu Einfachbindungen zu hydrieren vermögen. Sie enthalten in der Regel eines oder mehrere Elemente der I., VI., VII. oder VIII.-Nebengruppe des Periodensystems der Elemente, bevorzugt die Elemente Kupfer, Chrom, Molybdän, Mangan, Rhenium, Eisen, Ruthenium, Kobalt, Nickel, Platin und Palladium. Besonders bevorzugt verwendet man Katalysatoren, die mindestens ein Element ausgewählt aus Kupfer, Eisen, Nickel, Platin und Palladium enthalten.

Der Metallgehalt dieser Katalysatoren liegt in der Regel zwischen 0,1 bis 100 Gew.-%, bevorzugt 0,2 bis 95 Gew.-%, besonders bevorzugt 0,5 bis 95 Gew.-%.

Der Katalysator enthält bevorzugt zusätzlich mindestens ein Element ausgewählt aus den Elementen der II., III., IV. und VI.-Hauptgruppe, der II., III., IV. und V.-Nebengruppe des Periodensystems der Elemente und der Lanthanoiden als Promotor zur Aktivitätssteigerung.

Der Promotorgehalt des Katalysators beträgt in der Regel bis 25 Gew.-%, bevorzugt 0,001 - 15 Gew.-%, besonders bevorzugt 0,01 - 13 Gew.-%.

Als Katalysatoren können Fällungs-, Träger-, oder Raney-Typ-Katalysatoren verwendet werden, deren Herstellung beispielsweise in Ullmanns, Encyclopädie der technischen Chemie, 4. Auflage, 1977, Band 13, Seiten 558-665 beschrieben ist.

Als Trägermaterialien können Aluminiumoxide, Titanoxide, Zirkoniumdioxid, Siliciumdioxid, Tonerden, z. B. Montmorillonite, Silikate wie Magnesium- oder Aluminiumsilikate, Zeolithe sowie Aktivkohlen verwendet werden. Bevorzugte Trägermaterialien sind Aluminiumoxide, Titandioxide, Siliciumdioxid, Zirkoniumdioxid und Aktivkohlen. Selbstverständlich können auch Mischungen verschiedener Trägermaterialien als Träger für im erfindungsgemäßen Verfahren anwendbare Katalysatoren dienen.

Diese Katalysatoren können entweder als Katalysatorformkörper, beispielsweise als Kugeln, Zylinder, Ringe, Spiralen, oder in Form von Pulvern verwendet werden. Bevorzugt werden die Katalysatoren als Formkörper eingesetzt. Geeignete Katalysatoren für die Hydrierung sind beispielsweise aus DE-A 12 85 992, DE-A 25 36 273,
EP-A 177 912, EP-A 394 841, EP-A 394 842, US 5,068,468, DE -A 1 641 707 und EP-A 922 689 bekannt, wobei die aus EP-A 394 841 bekannten Katalysatoren mit einem Gehalt an 20 bis 75 Gew.-% Nickeloxid, 10 bis 75 Gew.-Zirkoniumdioxid und 5 bis 50 Gew.-% Kupferoxid besonders bevorzugt sind.

Weiterhin können Katalysatoren vom Raney-Typ, wie beispielsweise Raney-Nickel, Raney-Kupfer, Raney-Kobalt, Raney-Nickel/Molybdän, Raney-Nickel/Kupfer, Raney-Nickel/Chrom, Raney-Nickel/Chrom/Eisen oder Rhenium-Schwamm für die Stufe (III) des erfindungsgemäßen Verfahrens verwendet werden. Raney-Nickel/Molybdän-Katalysatoren können beispielsweise nach dem in der US-A 4, 153, 578 beschriebenen Verfahren hergestellt werden. Diese Katalysatoren werden aber auch beispielsweise von der Firma Degussa , 63403 Hanau, Deutschland vertrieben. Ein Raney-Nickel-Chrom-Eisen-Katalysator wird beispielsweise unter der Handelsbezeichnung Katalysator Typ 11 112 W^{®} von der Firma Degussa vertrieben.

Bei der Verwendung von Fäll- oder Trägerkatalysatoren werden diese vor Beginn der Reaktion bei 150 bis 500°C im Wasserstoff- bzw. Wasserstoff/Inertgas-Strom reduziert. Diese Reduktion kann direkt im Hydrier-Reaktor durchgeführt werden. Falls die Reduktion in einem separaten Reaktor durchgeführt wird, können die Katalysatoren vor dem Ausbau bei z. B. 30°C mit sauerstoffhaltigen Gasgemischen oberflächlich passiviert werden. Die passivierten Katalysatoren können in diesem Fall im Hydrier-Reaktor vor dem Einsatz in einem Stickstoff/Wasserstoffstrom bei z. B. 180°C aktiviert oder auch ohne Aktivierung eingesetzt werden.

Die Katalysatoren können im Festbett oder in Suspension eingesetzt werden. Wenn die Katalysatoren in Form eines Festbetts angeordnet sind, wird der Reaktor in Rieselfahrweise oder in einem aufwärts gerichteten Gleichstrom von Flüssigkeit und Gas betrieben.

Suspendierte Katalysatoren werden mit einer Korngröße im Allgemeinen von 0,1 bis 500 µm, bevorzugt 0,5 bis 200 µm, besonders bevorzugt 1 bis 100 µm eingesetzt.
Wird mit suspendierten Katalysatoren gearbeitet, so wird bei Verwendung von gepackten Blasensäulen ebenfalls mit einem aufwärts gerichteten Gleichstrom von Flüssigkeit und Gas so gearbeitet, dass die Flüssigkeit und nicht das Gas als kontinuierliche Phase vorliegt.

Das erfindungsgemäß bei Festbettkatalysatoren und bei im Reaktionsmedium suspendierten Katalysatoren in gepackten Blasensäulen einzuhaltende Verhältnis von das Reaktionsgefäß verlassender zu zugeführter Gasmenge lässt sich in einfacher Weise einstellen, indem man die entsprechende Menge Wasserstoff entweder als Frischgas dosiert, oder technisch bevorzugt, Kreisgas rückführt und nur den durch chemischen Verbrauch und Abgas bedingten Wasserstoffverlust durch Frischwasserstoff ergänzt.

Das molare Verhältnis von Frischwasserstoff zu 1,4-Butindiol beträgt mindestens 2 : 1, bevorzugt zwischen 2,01 bis 3 : 1. Der Frischwasserstoff wird bevorzugt vor der Reaktionszone zugegeben, es ist jedoch auch möglich, Wasserstoff an anderen Stellen, ggf. zusätzlich einzuspeisen, beispielsweise durch Einsteckrohre in den Katalysatorbereich und/oder bei Verwendung von mehreren Reaktoren, vor und/oder in den jeweiligen Reaktor. Sofern mit Hydrierung mit Kreisgas betrieben wird, beträgt das molare Verhältnis Wasserstoff (Summe aus Frisch- und Kreisgaswasserstoff) zur Summe aus 1,4-Butindiol, 1,4-Butendiol und 1,4-Butandiol mindestens 3 : 1, bevorzugt 4 : 1 bis 100 : 1, besonders bevorzugt 5 : 1 bis 70 : 1. Kreisgas kann über alle verwendeten Hydrierreaktoren verteilt sein, oder nur auf einzelne Stufen, wie z. B. auf die sogenannten Hauptreaktoren. Dabei kann nach jedem Reaktor ein Gas-, Flüssigabscheider angeordnet sein, aus dem Gas für das Kreisgas entnommen werden kann.

Das erfindungsgemäße Verfahren wird an Festbettkatalysatoren mit Kreisgasfahrweise durchgeführt, d.h. das den Reaktor verlassende Gas wird im Kreislauf, gegebenenfalls nach Ergänzung mit frischem Wasserstoff, über einen Verdichter in den Reaktor rückgeführt. Es ist möglich, die gesamte Kreisgasmenge oder eine Teilmenge davon über einen Treibstrahlverdichter zu führen. In dieser bevorzugten Ausführungsform wird der Kreisgasverdichter durch eine kostengünstige Düse ersetzt. Die Verdichtungsarbeit wird über die ebenfalls im Kreis geführte Flüssigkeit eingebracht. Die erforderliche Druckerhöhung der Flüssigkeit zum Betreiben des Treibstrahlverdichters beträgt etwa 3 bis 5 bar.

Für die Durchführung des erfindungsgemäßen Verfahrens mit einem im Reaktionsmedium suspendierten Katalysator sind Strahldüsenreaktoren, Rührkessel und Blasensäulen mit Packungen, welche eine Packungsoberfläche von mindestens 500, bevorzugt 1000 bis 2000 m²/m³ aufweisen, geeignet. Strahldüsenreaktoren können in verschiedenen Bauarten Anwendung finden, wenn sie durch einen ausreichend hohen Energieeintrag, der erfahrungsgemäß oberhalb von 2 kW/m³ liegt, den für die Erfindung wesentlichen hohen Stoffübergang von der Gasphase an die Flüssigkeit mit den suspendierten Katalysatorteilchen gewährleisten können. Besonders geeignet sind Strahldüsenreaktoren, die mit einem Impulsaustauschrohr ausgerüstet sind. Eine industriell verbreitete Bauform eines Strahldüsenreaktors ist beispielsweise der in der EP-A 0 419 419 beschriebene Reaktor. Bei Werten für den Energieeintrag von 3 bis 5 kW/m³ ist mit diesem Reaktor eine Abscheidung der Gasphase noch in einfachen Abscheidern möglich, ohne Zusatzapparate wie Schaumzentrifugen oder Zyklone, benutzen zu müssen.

Rührbehälter sind für die Durchführung des erfindungsgemäßen Verfahrens nur dann geeignet, wenn der Energieeintrag in einem Bereich von 2 bis 10 kW/m³ liegt.

Strahldüsenreaktoren mit suspendierten Katalysatoren benötigen volumenbezogene Energieeinträge von mehr als 2 kW/m³, bevorzugt 3 - 5 kW/m³.

Für die Ausführung des erfindungsgemäßen Verfahrens im großtechnischen Maßstab ist es bevorzugt, die Hydrierung in mindestens zwei Stufen (Haupt- und Nachhydrierung) durchzuführen, wobei bevorzugt mindestens ein sogenannter Hauptreaktor und mindestens ein sogenannter Nachreaktor eingesetzt wird.

In dem Hauptreaktor oder in den Hauptreaktoren wird bevorzugt der größte Teil der Umsetzung stattfinden. Üblicherweise liegt der Umsatz aller zu hydrierenden Doppel-und Dreifachbindungen im Hauptreaktor oder in den Hauptreaktoren zwischen 60 und 99,99 %, bevorzugt zwischen 80 und 99,99 %, besonders bevorzugt 90 bis 99,99 %. Mit zu hydrierenden Doppel- und Dreifachbindungen sind in dieser Anmeldung Verbindungen gemeint, die noch C-C-Dreifach oder Doppelbindungen sowie C-O-Doppel-bindungen enthalten, wobei Verbindungen, die C-O-Doppelbindung enthalten, z.B. Butyraldehyd und 4-Hydroxybutyraldehyd bzw. dessen Acetale sind. Dabei ist es besonders bevorzugt, den Umsatz im Hauptreaktor so hoch zu haben, dass in dem Nachreaktor oder den Nachreaktoren die Resthydrierung ohne Kühlung durchgeführt werden kann. Die Kühlung während der Haupthydrierung kann wie technisch üblich mit innenliegenden oder außenliegenden Wärmetauschern gewährleistet werden. Bei außenliegenden Wärmetauschern wird ein Teilstrom des Hydrieraustrags verwendet, der nach Abkühlung mit Frischzulauf vermischt wieder in die Hydrierung zurückgeführt wird. Dabei beträgt beispielsweise das Verhältnis Umlauf zu Zulauf 2 : 1 bis 50 : 1, bevorzugt 3 : 1 bis 30 : 1, besonders bevorzugt 5 : 1 bis 15 : 1.

Die Hydrierung in dem oder den Hauptreaktoren (Haupthydrierung) wird bei Temperaturen zwischen 30 und 300°C, bevorzugt zwischen 50 und 250°C und besonders bevorzugt zwischen 70 und 220°C durchgeführt. Wird die Hydrierung im Festbett durchgeführt, so liegt der Hydrierdruck in der Haupthydrierung zwischen 25 und 350 bar, bevorzugt zwischen 100 und 330 bar, besonders bevorzugt zwischen 150 und 300 bar. Werden suspendierte Katalysatoren verwendet, so liegt der Reaktionsdruck zwischen 2 und 150 bar, bevorzugt zwischen 5 und 100 bar, besonders bevorzugt zwischen 10 und 65 bar.

Die Hydrierung in dem oder den Nachreaktoren (Nachhydrierung) kann unter den gleichen Temperatur und Druckbedingungen ablaufen wie die für die Haupthydrierung beschriebenen.

Es ist jedoch auch möglich, die Nachhydrierung bei 10 bis 50°C höheren Temperaturen durchzuführen. Dabei ist die Eingangstemperatur des ersten Nachreaktors bevorzugt gleich der Ausgangstemperatur des Hauptreaktors. Soll die Eingangstemperatur des Nachreaktors höher oder niedriger der Ausgangstemperatur des Hauptreaktors liegen, so wird der Strom zum Nachreaktor zusätzlich aufgewärmt oder abgekühlt. Zum Abkühlen kann bevorzugt ein Teilstrom aus dem Umlaufstrom des Hauptreaktors nach dessen Abkühlung verwendet werden.

In einer bevorzugten Ausführungsform der Haupthydrierung in Festbettfahrweise wird die Hydrierung in mindestens zwei parallelen Reaktoren durchgeführt. Nach längerem Betrieb der Hydrierung wird durch die in der 1,4-Butindiolsynthese entstandenen Salzen, kolloidalen und festen Komponenten, der Hydrierkatalysator belegt und dies führt bei fest angeordneten Katalysatoren zu erhöhtem Differenzdruckanstieg und bei suspendierten Katalysatoren zu verminderter Katalysatoraktivität und Selektivität. Für ein wirtschaftliches Verfahren ist es, insbesondere bei der Verwendung von teuren Katalysatoren, unerlässlich, eine möglichst lange Katalysatorlebensdauer zu erzielen. Würde man nun die Katalysatoren austauschen müssen, nachdem sich ein erhöhter Druckverlust und/oder eine nachlassende Aktivität und Selektivität eingestellt hatte, wären die Katalysatorkosten zu hoch. Die Katalysatoren werden deshalb aus den Reaktoren ausgebaut, mit Wasser gewaschen und anschließend wiederverwendet. Hat man mehrere parallele Reaktoren zur Verfügung, kann die Hydrierung weiterlaufen, ohne dass der gesamte Prozess abgestellt werden muss.

Es hat sich nun überraschend gezeigt, dass die Katalysatoren eine höhere Lebensdauer haben, wenn das aus der 1,4-Butindiolsynthese erhaltende Produkt erfindungsgemäß zwischengepuffert wurde. Durch das erfindungsgemäße Verfahren wird die Zeit zwischen den Waschzyklen deutlich verlängert und die Katalysatoren können wesentlich häufiger nach dem Waschvorgang wiederverwendet werden. Ferner ist die Gesamtausbeute und die Reinheit des gewonnenen 1,4-Butandiol höher.

Es ist vorteilhaft, den pH während der Hydrierung zu kontrollieren. Für die pH-Wert-Einstellung können die gleichen sauer oder basisch wirkenden Stoffe wie sie vorstehend bei der Beschreibung Umsetzung von Acetylen und Formaldehyd (Stufe I) beschrieben wurden, verwendet werden. Der pH-Bereich der Hydrierung liegt im allgemeinen zwischen 5 und 11, bevorzugt zwischen 6 und 9, besonders bevorzugt zwischen 6,5 und 8. Die pH-Wert-Einstellung ist vorteilhaft, um einerseits die Lebensdauer der verwendeten Katalysatoren zu gewährleisten, da sich vor allem die Katalysatorträger im sauren oder basischen Bereich auflösen können, andererseits um Nebenreaktion zurückzudrängen. So ist beispielsweise die Bildung von 2-Methyl-1,4-butandiol auf die Reaktion von Formaldehyd und 4-Hydroxybutyraldehyd (einem Zwischenprodukt der Hydrierung) v.a. unter basischen Bedingungen zurückzuführen. Ferner verhindert die pH-Kontrolle Korrosion an Werkstoffen im Reaktor und Rohrleitungen.

In Ausführungsformen, in denen mindestens ein Nachreaktor verwendet wird, wird in diesem Nachreaktor der Restumsatz an zu hydrierenden, in der Haupthydrierung verbliebenen Doppel- und Dreifachbindungen angestrebt. Bevorzugt wird ein Umsatz von mindestens 99,95 %, besonders bevorzugt ist ein Umsatz von mindestens 99,99 % erreicht. Der Umsatz im Nachreaktor ist jedoch ganz besonders bevorzugt vollständig (100 %). Der Nachhydrierung im Nachreaktor oder in den Nachreaktoren wird bevorzugt an fest angeordneten Katalysatoren durchgeführt. Dabei kann Riesel- oder Aufwärtsfahrweise gewählt werden, wobei bevorzugt ohne Flüssigrückführung gearbeitet wird.

Da kolloidale und feste Verunreinigungen aus der Butindiolsynthese größtenteils in der Haupthydrierung niedergeschlagen werden, ist eine Waschung des Katalysators in der Nachhydrierung meist nicht notwendig, zumindest aber deutlich seltener.

Der aus der Nachhydrierung stammende Produktstrom wird in einen Abscheider geleitet, bei dem sich Gas- und Flüssigphase trennen. Die Gasphase enthält überwiegend Wasserstoff. Sofern mit Kreisgas hydriert wird, wird dieser Abscheider bevorzugt beim selben Druck betrieben wie die Hydrierung, damit das daraus entnommene Gas nicht noch zusätzlich komprimiert werden muss. Ein Teil des Gasstroms kann als Abgas entsorgt werden.

Die flüssige Phase des Abscheiders kann über eine Druckentspannung in einen weiteren Gas-, Flüssig-Abscheider oder direkt in eine Destillationseinheit oder mehrere Destillationseinheiten geleitet werden. In beiden Fällen wird gelöstes Gas, überwiegend Wasserstoff, aber auch Inertgase wie z.B. Stickstoff und Methan ausgeschleust und bevorzugt verbrannt, wobei Energie erzeugt werden kann. Es ist auch möglich, zuerst einen Abscheider und dann eine Destillationseinheit oder mehrere Destillationseinheiten zu verwenden. Der Druck nach der Entspannung liegt im Allgemeinen zwischen Normaldruck und 20 bar, bevorzugt zwischen Normaldruck und 15 bar, besonders bevorzugt zwischen Normaldruck und 10 bar. Der Flüssigaustrag aus dem Abscheider wird anschließend der destillativen Aufarbeitung zugeführt und wird im Folgenden mit Strom A bezeichnet und enthält im Allgemeinen Methanol, Propanol, Butanol, Wasser, gamma-Butyrolacton, 2-Methyl-1,4-butandiol, 1,4-Butandiol, ein Acetal aus 4-Hydroxy-butyraldehyd und 1,4-Butandiol, Acetal, Pentandiole wie z.B. 1,5-Pentandiol und 2-Methyl-1,5-Pentandiol, Salze, organische Hochsieder sowie weitere, mengenmäßig unbedeutende Nebenkomponenten.

Die Temperaturen der im Folgenden beschriebenen Destillationen werden durch die Dampfdrücke der in den Stoffströmen enthaltenen Komponenten und dem eingestellten Druck determiniert. Die Destillationen laufen bevorzugt wärmeintegriert, um möglichst wenig Energie zu verbrauchen.

Zur Auftrennung des Stromes A werden bevorzugt mehrere Destillationseinheiten verwendet. Beispielsweise werden Leichtsieder wie Methanol, Propanol, Butanol und Wasser bei Drücken (absolut) zwischen 0,5 und 20 bar, bevorzugt zwischen 0,8 und 10 bar vom 1,4-Butandiol-haltigen Produktstrom abgetrennt. Diese Abtrennung kann in einer Destillationseinheit oder in mehreren erfolgen. Bevorzugt ist, in einer Destillationseinheit ein Gemisch aus Methanol, Propanol und Butanol, das auch Wasser enthält, zuerst abzudestillieren und dann in einer weiteren Kolonne oder mehreren weiteren Kolonnen, die bevorzugt über eine Wärmeintegration verfügen, das restliche Wasser abzudestillieren. Der Methanol, Propanol und Butanol sowie Wasser enthaltende Produktstrom kann entweder verbrannt, oder separat in die einzelnen Komponenten getrennt werden, um sie beispielsweise als Lösemittel in anderen Prozessen zu verwenden. Methanol kann beispielsweise in der Formaldehydherstellung verwertet werden.
Das abgetrennte Wasser kann, wenn es ausreichend wenig organisches Material enthält, bevorzugt einer Abwasserbehandlung zugeführt werden.

Der nach der destillativen Abtrennung des Wassers und Leichtsiedern erhaltene Produktstrom wird im Folgenden B genannt und enthält gamma-Butyrolacton, 2-Methyl-1,4-butandiol, 1,4-Butandiol, Acetal, Pentandiole, Salze, organische Hochsieder sowie weitere, mengenmäßig unbedeutende Nebenkomponenten und wird weiter destillativ behandelt, wobei es mehrere Möglichkeiten hierzu gibt:
i) Strom B kann in flüchtige organische Bestandteile, im Folgenden Strom C, enthaltend gamma-Butyrolacton, 2-Methyl-1,4-butandiol, 1,4-Butandiol, Acetal, Pentandiole sowie weitere, mengenmäßig unbedeutende Nebenkomponenten, und überwiegend schwerflüchtige organische Bestandteile, die die anorganischen Komponenten enthalten, aufgetrennt werden. Dies wird üblicherweise bei einem Druck (absolut) von 0,005 bis 1 bar, bevorzugt zwischen 0,01 und 0,7 bar, besonders bevorzugt bei 0,02 und 0,4 bar beispielsweise in einem Fallfilmverdampfer oder einem Dünnschichtverdampfer durchgeführt. Die abgetrennten Hochsieder werden zusammen mit enthaltenen Salzen bevorzugt verbrannt.
   Strom C wird weiterhin destillativ aufgetrennt, wobei dies beispielsweise in einer Trennwandkolonne geschieht, bei der der Strom C auf der einen Seite der Trennwand in die Kolonne eingebracht wird, während auf der anderen Seite der Trennwand nahezu reines 1,4-Butandiol abgezogen wird. Über Kopf der Trennwandkolonne wird ein Gemisch bestehend aus überwiegend gamma-Butyrolacton mit 1,4-Butandiol und weiteren, mengenmäßig unbedeutenden Komponenten abgezogen. Dieses Kopfprodukt kann in Gänze z.B. über die Verbrennung entfernt werden oder teilweise oder in Gänze in den Prozess zurückgeführt werden. Dabei kann das enthaltene gamma-Butyrolacton der pH-Regulierung dienen. Bevorzugt wird dieser Kopfstrom in die Hydrierstufe zurückgeführt.
   Der Sumpfstrom der Trennwandkolonne enthält überwiegend 1,4-Butandiol und weitere Produkte wie 2-Methyl-1,4-butandiol, Acetal sowie mengenmäßig unbedeutende Komponenten und kann in Gänze z.B. über die Verbrennung entfernt werden oder teilweise oder in Gänze in den Prozess zurückgeführt werden, beispielsweise dem Stoffstrom A oder B zugesetzt werden. Das nahezu reine 1,4-Butandiol kann als solches bereits verkaufsfähige Qualität besitzen, es kann aber auch noch in einer weiteren Kolonne feingereinigt werden, um 1,4-Butandiol-Reinheiten von > 99,5 % zu erhalten. Neben einer Trennwandkolonne sind auch mindestens zwei Kolonnen mit jeweils Kopf- und Sumpfabzug, aber auch mit Seitenabzug möglich. Die Drücke der Trennwandkolonne oder der alternativen Kolonnen zur Aufreinigung des Stroms C bzw. zur Feinreinigung des 1,4-Butandiols liegen zwischen 0,005 und 0,8 bar, bevorzugt zwischen 0,01 und 0,5 bar, besonders bevorzugt zwischen 0,02 und 0,2 bar.
ii) Strom B kann weiterhin destillativ aufgetrennt, wobei dies beispielsweise in mindestens zwei Kolonnen oder in einer Trennwandkolonne geschieht, bei der der Strom B auf der einen Seite der Trennwand in die Kolonne oder bei Verwendung von zwei Kolonnen in die erste eingebracht wird, während auf der anderen Seite der Trennwand bzw. bei Verwendung von zwei Kolonnen aus der zweiten Kolonne über Seitenabzug oder über Kopf nahezu reines 1,4-Butandiol abgezogen wird. Über Kopf der Trennwandkolonne bzw. bei Verwendung von zwei Kolonnen aus der ersten wird ein Gemisch bestehend aus überwiegend gamma-Butyrolacton mit 1,4-Butandiol und anderen mengenmäßig unbedeutenden Komponenten abgezogen. Dieses Kopfprodukt kann in Gänze z.B. über die Verbrennung entfernt werden oder teilweise oder in Gänze in den Prozess zurückgeführt werden. Dabei kann das enthaltene gamma-Butyrolacton der pH-Regulierung dienen. Bevorzugt wird dieser Kopfstrom in die Hydrierstufe zurückgeführt.
   Der Sumpfstrom der Trennwandkolonne bzw. bei Verwendung von zwei Kolonnen der Sumpfstrom der zweiten Kolonne enthält überwiegend 1,4-Butandiol, Salze, Hochsieder und weitere Produkte wie 2-Methyl-1,4-butandiol, Acetal sowie mengenmäßig unbedeutende Komponenten und kann in Gänze z.B. über die Verbrennung entfernt werden oder bevorzugt z.B. in einem Dünnschichtverdampfer oder in einem Dünnschichtverdampfer in Hochsieder und Salze, sowie einen 1,4-Butandiol enthaltenden Strom, der z.B. Strom A oder B zugemischt werden kann, aufgetrennt werden. Das nahezu reine 1,4-Butandiol kann als solches bereits verkaufsfähige Qualität besitzen, es kann aber auch noch in einer weiteren Kolonne feingereinigt werden, um 1,4-Butandiol-Reinheiten von > 99,5 % zu erhalten. Die Drücke (absolut) der Trennwandkolonne zur Aufreinigung des Stroms B bzw. zur Feinreinigung des 1,4-Butandiols liegen zwischen 0,005 und 0,8 bar, bevorzugt zwischen 0,01 und 0,5 bar, besonders bevorzugt zwischen 0,02 und 0,2 bar. Die Drücke (absolut) des Fallfilmverdampfers oder des Dünnschichtverdampfers liegen bei 0,005 bis 1 bar, bevorzugt zwischen 0,01 und 0,7 bar, besonders bevorzugt zwischen 0,02 und 0,4 bar.
iii) Strom B kann in einer Kolonne so aufgetrennt werden, dass über Kopf ein Produktstrom abgezogen wird bestehend aus überwiegend gamma-Butyrolacton, daneben noch 1,4-Butandiol und andere, mengenmäßig unbedeutende Komponenten. Dieses Kopfprodukt kann in Gänze z.B. über die Verbrennung entfernt werden oder teilweise oder in Gänze in den Prozess zurückgeführt werden. Dabei kann das enthaltene gamma-Butyrolacton der pH-Regulierung dienen. Bevorzugt wird dieser Kopfstrom in die Hydrierstufe zurückgeführt. Der Sumpfstrom, im Folgenden als Strom D bezeichnet, enthält 2-Methyl-1,4-butandiol, 1,4-Butandiol, Acetal sowie weitere, mengenmäßig unbedeutende Nebenkomponenten) und schwerflüchtige organische Bestandteile sowie Salze. Dieser Strom D kann beispielsweise in einem Fallfilmverdampfer oder Dünnschichtverdampfer bei Drücken (absolut) zwischen 0,005 bis 1 bar, bevorzugt zwischen 0,01 und 0,7 bar, besonders bevorzugt zwischen 0,02 und 0,4 bar in Hochsieder, die schwerflüchtige organische Bestandteile und Salze enthalten sowie einen überwiegend 1,4-Butandiol enthaltenden Strom aufgetrennt werden. Der 1,4-Butandiol enthaltende Strom kann in einer weiteren Kolonne, die als Trennwandkolonne oder als normale Seitenabzugskolonne ausgeführt sein, wobei reines 1,4-Butandiol in beiden Fällen als Seitenabzug entnommen wird, aufgetrennt werden. Die Drücke der Trennwandkolonne bzw. Seitenabzugskolonne liegen zwischen 0,005 und 0,8 bar, bevorzugt zwischen 0,01 und 0,5 bar, besonders bevorzugt zwischen 0,02 und 0,2 bar. Die Kopf- und Sumpfsströme können ganz oder teilweise entsorgt oder ganz oder teilweise beispielsweise in die Ströme A, B und/oder D zurückgeführt werden und enthalten überwiegend 1,4-Butandiol.
iiii) Strom D kann so aufgetrennt werden, dass in einer Kolonne, die als Trennwandkolonne oder als normale Seitenabzugskolonne ausgeführt sein kann, reines 1,4-Butandiol in beiden Fällen als Seitenabzug entnommen wird. Die Drücke der Trennwandkolonne bzw. Seitenabzugskolonne liegen zwischen 0,005 und 0,8 bar, bevorzugt zwischen 0,01 und 0,5 bar, besonders bevorzugt zwischen 0,02 und 0,2 bar. Der Kopfstrom kann ganz oder teilweise entsorgt oder ganz oder teilweise beispielsweise in die Ströme A und/oder B zurückgeführt werden und enthält überwiegend 1,4-Butandiol. Der Sumpfstrom, der noch 1,4-Butandiol, Hochsieder und Salze enthält, kann beispielsweise in einem Fallfilmverdampfer oder Dünnschichtverdampfer bei Drücken (absolut) zwischen 0,005 bis 1 bar, bevorzugt zwischen 0,01 und 0,7 bar, besonders bevorzugt bei 0,02 und 0,4 bar in Hochsieder, die schwerflüchtige organische Bestandteile und Salze enthalten sowie einen überwiegend 1,4-Butandiol enthaltenden Strom, der beispielsweise den Strömen A und/oder B und/oder D beigemischt wird, aufgetrennt werden.
iiiii) Strom B kann in einer Kolonne so aufgetrennt werden, dass über Kopf ein Strom E bestehend aus überwiegend 1,4-Butandiol, das noch gamma-Butyrolacton, 2-Methyl-1,4-butandiol und Acetal enthält, und über Sumpf ein Strom F, der neben 1,4-Butandiol, 2-Methyl-1,4-butandiol, Acetal auch die Hochsieder und Salze enthält, erhalten wird. Der Kopfstrom E wird in einer weiteren Kolonne aufdestilliert, wobei als Kopfstrom überwiegend gamma-Butyrolacton mit 1,4-Butandiol und anderen, mengenmäßig unbedeutenden Komponenten erhalten wird. Dieses Kopfprodukt kann in Gänze z.B. über die Verbrennung entfernt werden oder teilweise oder in Gänze in den Prozess zurückgeführt werden. Dabei kann das enthaltene gamma-Butyrolacton der pH-Regulierung dienen. Bevorzugt wird dieser Kopfstrom in die Hydrierstufe zurückgeführt. Über einen Seitenstrom wird reines 1,4-Butandiol erhalten, kann aber auch als Sumpfprodukt abgezogen werden. Der Seitenabzug kann dabei flüssig oder gasförmig sowohl im Verstärkungs als auch im Abtriebsteil oder genau in der Mitte der Kolonne erfolgen. Die Kolonne verfügt über eine theoretische Bodenzahl zwischen 30 und 200, bevorzugt 50 bis 150. Der Sumpfstrom, der überwiegend 1,4-Butandiol enthält, kann als Reinprodukt abgezogen werden oder kann beispielsweise den Strömen A, B und/oder F zugeführt werden. Der Strom F kann in einer weiteren Kolonne aufgetrennt werden, wobei als Kopfstrom ein Gemisch anfällt, das überwiegend 1,4-Butandiol enthält, welches entweder in Gänze der Verbrennung zugeführt wird oder teilweise oder in Gänze z.B. dem Strom A, B und/oder E zugeführt werden kann. Als Sumpfstrom fällt ein Strom an, der neben 1,4-Butandiol auch die Hochsieder und die Salze enthält. Dieser Sumpfstrom wird vorzugsweise in einem Fallfilmverdampfer oder Dünnschichtverdampfer bei Drücken (absolut) zwischen 0,005 bis 1 bar, bevorzugt zwischen 0,01 und 0,7 bar, besonders bevorzugt zwischen 0,02 und 0,4 bar in Hochsieder, die schwerflüchtige organische Bestandteile und Salze enthalten sowie einen überwiegend 1,4-Butandiol enthaltenden Strom, der beispielsweise den Strömen A, B, D und/oder E und/oder F beigemischt wird, aufgetrennt werden. Die anfallenden Hochsieder werden zusammen mit den Salzen verbrannt.

Die in den Destillationsschritten verwendeten Kolonnenarten sind dem Fachmann gemeinhin bekannt. Es sind beispielsweise Packungskolonnen, Bodenkolonnen mit Siebböden, dual flow-Böden, Glöckenböden, Ventilböden usw.
Die Vakuumanlage bzw. Anlagen können mit verschiedenen Medien betrieben werden so z.B. Wasser. Es hat sich als vorteilhaft erwiesen, sie mit 1,4-Butandiol zu betreiben.

Das nach den vorstehenden Verfahrensvarianten erhaltende reine 1,4-Butandiol weist üblicherweise Reinheiten von > 99,5 %, typisch sind > 99,7 % auf. Wesentliche Begleitkomponenten sind dabei noch 2-Methy-1,4-butandiol und Acetal. Da v.a. das Acetal bei den typischen 1,4-Butandiolanwendungen stört, da es nur eine OH-Gruppe trägt, kann es sinnvoll sein, diese Komponente weiter abzureichern. Dies kann beispielsweise dadurch erreicht werden, dass man beispielsweise einen der Ströme C oder E oder bereits gereinigtes 1,4-Butandiol mit etwas Wasser versetzt und an einem Ni-haltigen Katalysator hydriert. Die Verfahrensweise ist beispielsweise in
WO 97/36846 beschrieben. Das Reaktionsprodukt kann nach Wasserabtrennung an geeigneter Stelle im Verfahren, z.B. als Strom C oder E wieder in das Verfahren eingebracht werden. Im Prinzip wird bei dieser Vorgehensweise der Strom C oder E durch eine Hydrierstufe unterbrochen. Durch dieses Vorgehen, lässt sich besonders acetalarmes 1,4-Butandiol erzeugen. Es enthält dann typischerweise weniger als 500 ppm Acetal.

Es hat sich als vorteilhaft erwiesen, dass, eine besonders hohe 1,4-Butandiolreinheit erhalten werden kann, wenn bei den Vakuumdestillationen möglichst wenig Sauerstoff zugegen ist. Dabei bedeutet besonders hohe Reinheit, dass möglichst wenig monofunktionelle Komponenten und Aldehydderivate wie Acetal, 4-Hydroxybutyraldehyd oder dessen cyclisches Halbacetals und gamma-Butyrolacton enthalten sind. Das molare Verhältnis Sauerstoff zu 1,4-Butandiol in den Vakuumkolonnen übersteigt erfindungsgemäß1 : 500 nicht. Bevorzugt ist das Verhältnis unter 1 : 1000, besonders bevorzugt unter 1 : 1500, insbesondere bevorzugt unter 1: 2500.

Die erfindungsgemäßen Sauerstoff zu Butandiolverhältnisse erreicht man dadurch, dass man beim Aufbau und Betrieb der Kolonne oder Destillationseinheit den Verbleib bzw. das Eindringen von Sauerstoff vermeidet. Erfindungsgemäß wurde erkannt, dass, bevorzugt in der Kolonne, in der das 1,4-Butandiol als Reinprodukt gewonnen werden soll, ein erfindungsgemäßes Sauerstoff zu 1,4-Butandiol-Verhältnis eingehalten werden muss.

Die erfindungemäßen Sauerstoff -Butandiol-Molverhältnisse werden durch sorgfältige Abdichtungen der Kolonnen mit Hilfe von Nut- oder Federdichtungen; Verwendung von Dichtungsmassen wie Silikondichtmassen, Vermeidung von Flanschen, wie sie üblicherweise für die Temperatur oder Druckmessstellen an Kolonnen eingesetzt werden, Verwendung von gekammerten Kolonnen oder Destillationseinheiten mit Begasung der Kammer mit Inertgasen wie zum Beispiel Argon oder Stickstoff, eingehalten.

Die Menge des Sauerstoffs, die in die Kolonne eingetragen wird, kann beispielsweise vor Inbetriebnahme des Zulaufstromes durch Messung der Menge des Abgasstromes und dessen Sauerstoffgehaltes nach dem Vakuumaggregat jeder Vakuumkolonne, beispielsweise durch Gaschromatographie, bestimmt werden. Während des Betriebes ist zu beachten, dass der Sauerstoffgehalt zu niedrig angezeigt werden könnte, da Sauerstoff unter diesen Bedingungen bereits abreagieren kann. Einen wichtigen Hinweis kann in diesem Zusammenhang Verhältnis Sauerstoff zu Stickstoff geben, das dem der Umgebungsluft entsprechen sollte. Eine weitere Möglichkeit der Bestimmung des Sauerstoffgehaltes ist, die Kolonne ohne Produktzulauf zu evakuieren, die Kolonne vom Vakuumaggregat durch Schließen eines Ventils zu trennen und den Anstieg des Druckes pro Zeiteinheit in der Kolonne zu beobachten. Bei Kenntnis des Kolonnenvolumens ergibt sich daraus leicht die Menge des Sauerstoffeintrags pro Zeiteinheit.

Das nach den vorstehenden Verfahrensvarianten erhaltende 1,4-Butandiol weist üblicherweise Reinheiten von > 99,5 %, typisch sind > 99,8 % auf. Wesentliche Begleitkomponente ist dabei noch 2-Methyl-1,4-butandiol, das als Monoalkohol besonders unerwünschte Acetal liegt im Allgemeinen unter 0,1 %, im Regelfall unter 0,07 % vor.

1,4-Butandiol findet in der Technik in großen Mengen Anwendung zum Beispiel bei der THF-Herstellung oder als Diolkomponente in Polyestern.

### Beispiele:

In den Beispielen sind die angegebenen Drücke immer absolut, die Analytik der Hydrierausträge und des Reinbutandiols ist in GC-Flächenprozenten angegeben.

### Beispiel 1

In einer Reaktorkaskade, bestehend aus 3 zylindrischen 10 m langen Reaktoren mit einem Durchmesser von 15 cm, gefüllt mit einem Katalysator (ca. 15% CuO, ca. 4% Bi₂O3 auf SiO₂) in Form von 0,5 - 2 mm Splitt, hergestellt nach DE-A 26 02 418 , die jeweils sowohl mit Kreisgas als auch mit Flüssigumlauf in Aufwärtsfahrweise betrieben wurden (Umlauf zu Zulauf 10:1), wurden 20 kg/h 32 %iger, wässriger Formaldehyd und 2,8 kg/h Acetylen bei 5 bar und 70 bis 90°C bei einem pH von 6 umgesetzt. Das Reaktionsprodukt des ersten Reaktors wurde in den zweiten und das des zweiten in den dritten Reaktor gefördert. Auf diese Weise wurden > 95 % des Formaldehyds und des Acetylens zu 1,4-Butindiol umgesetzt. Der pH der Reaktion wurde so gesteuert, dass nach jedem Reaktor der pH gemessen wurde und bei Bedarf kleine Mengen 1 %iger wässriger NaOH-Lösung zudosiert wurden. Der Reaktionsaustrag des dritten Reaktors wurde in einem Abscheider in Gas- und Flüssigphase getrennt. Die Flüssigphase enthielt ca. 50 Gew.-% Butindiol, 1,3 Gew.-% Propinol 0,5 Gew.-% Formaldehyd,
0,5 Gew.-% Methanol, gelöstes Acetylen und mehrere 100 ppm nicht flüchtiger Oligomerer, Polymere und Katalysatorbestandteile sowie < 0,5 % andere Verunreinigungen sowie Wasser. Die Gasphase, die im wesentlichen Acetylen enthielt, wurde überwiegend als Kreisgas zurückgeführt, 1 % des Gasstrome wurde ausgeschleust. Der flüssige Austrag des Abscheiders wurde in eine Kolonne geleitet, bei der über Kopf bei 0,2 bar absolut und 90°C Sumpftemperatur Wasser, Formaldehyd, Methanol und Propinol abgetrennt (ca. 1 kg) und in die Reaktion zurückgeführt werden.

Der Sumpfaustrag wurde kontinuierlich in einen Zwischenpuffer geleitet, bei dem die mittlere Verweilzeit 10 h bei 60°C und 1 bar (absolut) betrug. Aus diesem Zwischenpuffer wurde die Butindiol-haltige Lösung entnommen und in einer zweistufigen Reaktorkaskade mit Wasserstoff an einem Ni-Katalysator gemäß EP-A 394 841 in Form von 3 x 3 mm Tabletten (ca. 38 Gew.-% Ni, ca. 12 Gew.-% Cu auf ZrO2/MoO3) hydriert. Das molare Verhältnis Frischwasserstoff zu 1,4-Butindiol betrug dabei 2,1 zu 1. Der erste Hydrierreaktor (10 m Länge, 10 cm Durchmesser) wurde mit Flüssigumlauf zur Kühlung in Aufwärtsfahrweise bei 250 bar Reaktoreingangsdruck und 120 - 140°C betrieben. Zur Einstellung des pH auf ca. 7,2 wurde dem Zulauf 1 %ige wässrige Na-OH bzw. gamma-Butyrolacton zudosiert. Der zweite Reaktor (10 m Länge, 5 cm Durchmessser) wurde in Rieselfahrweise von 140 - 160 bis 140 bis 175°C bei 250 bar betrieben. Der Austrag wurde in einem Abscheider in Flüssigphase und Gasphase getrennt, wobei die Gasphase mittels Kreisgaskompressor zurückgeführt wurde. Aus dem Gasstrom wurden kontin. ca. 1 % der Menge des Frischgases als Abgas ausgeschleust und verbrannt. Der Hydrieraustrag wurde auf ca. 5 bar entspannt und der dabei freiwerdende Gasstrom wurde ebenfalls der Verbrennung zugeführt. Anschließend wurde der entgaste Hydrieraustrag in eine Kaskade von Kolonnen in die einzelnen Bestandteile aufgetrennt. In einer ersten Kolonne wurden Leichtsieder wie Methanol, Propanol und n-Butanol zusammen mit Wasser bei ca. 5 bar und einer Sumpftemperatur von ca. 170°C über Kopf abgetrennt und der Verbrennung zugeführt. Der Sumpfstrom gelangte in eine zweite Kolonne, bei der ebenfalls bei ca. 0,3 bar und ca. 130°C Sumpftemperatur ganz überwiegend Wasser über Kopf abdestilliert wurde. Der Sumpfstrom der zweiten Kolonne wurde in einer dritten Kolonne bei ca. 0,15 bar und ca. 175°C Sumpftemperatur so aufgetrennt, dass über Kopf überwiegend 1,4-Butandiol zusammen mit gamma-Butyrolacton, 2-Methyl-1,4-butandiol, Acetal, Pentandiolen und einige weitere, mengenmäßig nicht bedeutenden Komponenten abdestilliert wurden. Dieser Kopfstrom wurde in einer vierten Kolonne, die bei ca. 0,04 bar und ca. 165°C Sumpftemperatur betrieben wurde, in einen Kopfstrom, der neben 1,4-Butandiol überwiegend gamma-Butyrolacton enthielt, einen Seitenstrom, der überwiegend aus 1,4-Butandiol bestand und einen Sumpfstrom, der ebenfalls aus überwiegend 1,4-Butandiol bestand und in den Sumpfstrom der dritten Kolonne eingespeist wurde aufgetrennt. Der Sumpfstrom der dritten Kolonne wurde zusammen mit dem der vierten Kolonne in einer fünften Kolonne bei ca. 0,05 bar und 170°C Sumpftemperatur so aufgetrennt, dass der Kopfstrom, der überwiegend 1,4-Butandiol enthielt in den Zulauf der dritten Kolonne zurückgeführt wurde, während der Sumpfstrom, der neben wenig 1,4-Butandiol Hochsieder und Salze enthielt, ausgeschleust und verbrannt wurde.

Verlauf der Hydrierung sowie Reinheit des 1,4-Butandiols nach Aufreinigung

Nach 24 h wurde im Hydrieraustrag des ersten Reaktors (wasserfrei gerechnet) ca. 94 % 1,4-Butandiol, 0,1 % 1,4-Butendiol, 0,05 % gamma-Butyrolacton, 0,05 % 2-Methyl-1,4-butandiol, 1,5 % Methanol, 2,5 % n-Propanol, 1,1 % n-Butanol, 0,07 % Acetal, 0,1 % Pentandiole sowie eine Vielzahl, mengenmäßig untergeordnete Komponenten gefunden. Der Druckverlust (Druck Reaktorausgang minus Druck Reaktoreingang) lag bei 2,5 bar. Im Ausgang des Nachhydrierreaktors wurden (wasserfrei gerechnet) ca. 94,2 % 1,4-Butandiol, 0,04 % gamma-Butyrolacton, 0,06 % 2-Methyl-1,4-butandiol, 1,6 % Methanol, 2,5 % n-Propanol, 1,2 % n-Butanol, 0,04 % Acetal sowie eine Vielzahl, mengenmäßig untergeordnete Komponenten gefunden.

Das reine 1,4-Butandiol hatte die Zusammensetzung 99,90 % 1,4-Butandiol, 0,05 % 2-Methyl-1,4-butandiol, 0,04 % Acetal sowie mehrere, mengenmäßig unbedeutende Komponenten.

Nach 12 Wochen Betriebszeit der Hydrierung fanden sich im Hydrieraustrag des ersten Reaktors (wasserfrei gerechnet) ca. 91 % 1,4-Butandiol, 1,1 % 1,4-Butendiol, 0,05 % 1,4-Butindiol, 0,08 % gamma-Butyrolacton, 0,08 % 2-Methyl-1,4-butandiol, 1,5 % Methanol, 2,3 % n-Propanol, 2,9 % n-Butanol, 0,15 % Acetal sowie eine Vielzahl, mengenmäßig untergeordnete Komponenten. Der Druckverlust (Druck Reaktorausgang minus Druck Reaktoreingang) lag bei 4,5 bar. Im Ausgang des zweiten Hydrierreaktors fanden sich (wasserfrei gerechnet) ca. 91,2 % 1,4-Butandiol, 0,05 % gamma-Butyrolacton, 0,09 % 2-Methyl-1,4-butandiol, 1,7 % Methanol, 2,5 % n-Propanol, 3,0 % n-Butanol, 0,13 % Acetal sowie eine Vielzahl, mengenmäßig untergeordnete Komponenten.

Das reine 1,4-Butandiol hatte die Zusammensetzung 99,80 % 1,4-Butandiol, 0,08 % 2-Methyl-1,4-butandiol, 0,1 % Acetal sowie mehrere, mengenmäßig unbedeutende Komponenten.

Nach diesen 12 Wochen Betriebszeit wurde der erste Hydrierreaktor mit Wasser gespült und der Katalysator anschließend unter Wasser ausgebaut und mit Wasser von anhaftenden Verunreinigungen freigewaschen und anschließend wieder eingebaut. Danach stellte sich nahezu der selbe Verlauf bei Hydrierung und 1,4-ButandiolReinheit ein wie zuvor. Es konnten insgesamt 4 Waschzyklen durchlaufen werden, bis das Hydrierergebnis langsam schlechter wurde.

### Vergleichsbeispiel 1

Beispiel 1 wurde wiederholt, mit dem Unterschied, dass der Austrag aus der Umsetzung von Acetylen mit Formaldehyd nicht zwischengepuffert wurde, sondern gleich in der Hydrierung umgesetzt wurde. Es stellte sich zu Beginn alles so ein wie im erfindungsgemäßen Beispiel, allerdings wurden diesmal die Werte wie im erfindungsgemäßen Beispiel nach 12 Wochen, bereits nach 8 Wochen erreicht (Hydrieraustrag des ersten Reaktors (wasserfrei gerechnet) ca. 91 % 1,4-Butandiol, 1,1 % 1,4-Butendiol, 0,06 % 1,4-Butindiol, 0.08 % gamma-Butyrolacton, 0,08 % 2-Methyl-1,4-butandiol,
1,4 % Methanol, 2,3 % n-Propanol, 2,9 % n-Butanol, 0,16 % Acetal sowie eine Vielzahl, mengenmäßig untergeordnete Komponenten. Der Druckverlust (Druck Reaktorausgang minus Druck Reaktoreingang) lag bei 4,6 bar. Im Ausgang des zweiten Hydrierreaktors fanden sich (wasserfrei gerechnet) ca. 91,2 % 1,4-Butandiol, 0,05 % gamma-Butyrolacton, 0,09 % 2-Methyl-1,4-butandiol, 1,6 % Methanol, 2,5 % n-Propanol, 3,0 % n-Butanol, 0,14 % Acetal sowie eine Vielzahl, mengenmäßig untergeordnete Komponenten. Das reine 1,4-Butandiol hatte die Zusammensetzung 99,80 % 1,4-Butandiol, 0,08 % 2-Methyl-1,4-butandiol, 0,1 % Acetal sowie mehrere, mengenmäßig unbedeutende Komponenten).

Nach Ausbau, Waschung und Wiedereinbaus des Hydrierkatalysators wurden die Ausgangswerte nicht mehr ganz erreicht, so dass in der zweiten Phase bereits nach 7 Wochen der Katalysator wieder gewaschen werden musste.

### Beispiel 2

Beispiel 1 wurde wiederholt, aber mit folgenden Änderungen: Zwischenpufferung 2 Stunden bei 75°C; Hydrierkatalysator mit der Zusammensetzung ca. 56 % Ni auf SiO2/Al2O3.

Nach 24 h wurde im Hydrieraustrag des ersten Reaktors (wasserfrei gerechnet)
ca. 93,5 % 1,4-Butandiol, 0,3 % 1,4-Butendiol, 0,05 % gamma-Butyrolacton, 0,05 % 2-Methyl-1,4-butandiol, 1,5 % Methanol, 2,5 % n-Propanol, 1,1 % n-Butanol, 0,1 % Acetal, 0,1 % Pentandiole sowie eine Vielzahl, mengenmäßig untergeordneter Komponenten gefunden. Der Druckverlust (Druck Reaktorausgang minus Druck Reaktoreingang) lag bei 2,4 bar. Im Ausgang des Nachhydrierreaktors wurden (wasserfrei gerechnet) ca. 94,0 % 1,4-Butandiol, 0,04 % gamma-Butyrolacton, 0,06 % 2-Methyl-1,4-butandiol, 1,4 % Methanol, 2,1 % n-Propanol, 1,3 % n-Butanol, 0,06 % Acetal sowie eine Vielzahl, mengenmäßig untergeordneter Komponenten gefunden.

Das reine 1,4-Butandiol hatte die Zusammensetzung 99,87 % 1,4-Butandiol, 0,05 % 2-Methyl-1,4-butandiol, 0,07 % Acetal sowie mehrere, mengenmäßig unbedeutende Komponenten.

Nach 10 Wochen Betriebszeit der Hydrierung fanden sich im Hydrieraustrag des ersten Reaktors (wasserfrei gerechnet) ca. 90,5 % 1,4-Butandiol, 1,3 % 1,4-Butendiol, 0,07 % 1,4-Butindiol, 0,06 % gamma-Butyrolacton, 0,09 % 2-Methyl-1,4-butandiol, 1,5 % Methanol, 2,2 % n-Propanol, 2,9 % n-Butanol, 0,16 % Acetal sowie eine Vielzahl, mengenmäßig untergeordneter Komponenten. Der Druckverlust (Druck Reaktorausgang minus Druck Reaktoreingang) lag bei 4,6 bar. Im Ausgang des zweiten Hydrierreaktors fanden sich (wasserfrei gerechnet) ca. 92,2 % 1,4-Butandiol, 0,05 % gamma-Butyrolacton, 0,09 % 2-Methyl-1,4-butandiol, 1,5 % Methanol, 2,5 % n-Propanol, 3,0 % n-Butanol, 0,14 % Acetal sowie eine Vielzahl, mengenmäßig untergeordnete Komponenten.

Das reine 1,4-Butandiol hatte die Zusammensetzung 99,80 % 1,4-Butandiol, 0,08 % 2-Methyl-1,4-butandiol, 0,11 % Acetal sowie mehrere, mengenmäßig unbedeutende Komponenten.

Nach diesen 10 Wochen Betriebszeit wurde der erste Hydrierreaktor mit Wasser gespült und der Katalysator anschließend unter Wasser ausgebaut und mit Wasser von anhaftenden Verunreinigungen freigewaschen und anschließend wieder eingebaut. Danach stellte sich nahezu der selbe Verlauf bei Hydrierung und 1,4-ButandiolReinheit ein wie zuvor. Es konnten insgesamt 5 Waschzyklen durchlaufen werden, bis das Hydrierergebnis langsam schlechter wurde.

### Vergleichsbeispiel 2

Für die Aufarbeitung wurde technisches 1,4-Butandiol in Form einer 54 Gew.-%igen wässrigen Lösung eingesetzt, das durch Hydrierung von technischem 1,4-Butindiol an einem Ni-Katalysator gemäß Beispiel 1 der EP-A 482 445 gewonnen wurde und die folgende Zusammensetzung aufwies (wasserfrei gerechnet): ca. 94 % 1,4-Butandiol, 0,05 % gamma-Butyrolacton, 0,6 % 2-Methyl-1,4-butandiol, 1,5 % Methanol, 2,3 % n-Propanol, 1,1 % n-Butanol, 0,07 % Acetal, 0,1 % Pentandiole sowie eine Vielzahl, mengenmäßig untergeordneter Komponenten.

Der Hydrieraustrag wurde in eine Kaskade von Kolonnen in die einzelnen Bestandteile aufgetrennt. In einer ersten Kolonne wurden Leichtsieder wie Methanol, Propanol und n-Butanol zusammen mit Wasser bei ca. 5 bar und einer Sumpftemperatur von ca. 170°C über Kopf abgetrennt und der Verbrennung zugeführt. Der Sumpfstrom gelangte in eine zweite Kolonne, bei der bei ca. 0,3 bar und ca. 130°C Sumpftemperatur ganz überwiegend Wasser über Kopf abdestilliert wurde. Der Sumpfstrom der zweiten Kolonne wurde in einer dritten Kolonne bei ca. 0,15 bar und ca. 175°C Sumpftemperatur so aufgetrennt, dass über Kopf überwiegend 1,4-Butandiol zusammen mit gamma-Butyrolacton, 2-Methyl-1,4-butandiol, Acetal, Pentandiolen und einige weitere, mengenmäßig nicht bedeutenden Komponenten abdestilliert wurden. Dieser Kopfstrom wurde in einer vierten Kolonne, die bei ca. 0,04 bar und ca. 165 °C Sumpftemperatur betrieben wurde, in einen Kopfstrom, der neben 1,4-Butandiol überwiegend gamma-Butyrolacton enthielt, einen Seitenstrom, der aus reinem 1,4-Butandiol bestand und einen Sumpfstrom, der ebenfalls aus überwiegend 1,4-Butandiol bestand und in den Sumpfstrom der dritten Kolonne eingespeist wurde aufgetrennt. Der Sumpfstrom der dritten Kolonne wurde zusammen mit dem der vierten Kolonne in einer fünften Kolonne bei ca. 0,05 bar und 170°C Sumpftemperatur so aufgetrennt, dass der Kopfstrom, der überwiegend 1,4-Butandiol enthielt, in den Zulauf der dritten Kolonne zurückgeführt wurde, während der Sumpfstrom, der neben wenig 1,4-Butandiol Hochsieder und Salze enthielt, ausgeschleust und verbrannt wurde.

Das reine 1,4-Butandiol hatte die Zusammensetzung 99,5 % 1,4-Butandiol, 0,1 % 2-Methyl-1,4-butandiol, 0,14 % Acetal, 0,05 % 4-Hydroxybutyraldehyd bzw. dessen cyclisches Halbacetal, 0,09 % gamma-Butyrolacton sowie mehrere weitere, mengenmäßig unbedeutende Komponenten.

Die vierte Kolonne der Kaskade, aus der das 1,4-Butandiol rein gewonnen wurde, verfügte über eine Messung des Abgasstroms und des darin enthaltenen Sauerstoffgehaltes. Es wurden im Abgasstrom durch GC-Analyse des Abgasstroms der Vakuumpumpe solche Mengen Sauerstoff gefunden , die darauf hinweisen, dass in der Kolonne mindestens ein molares Verhältnis von Sauerstoff zu 1,4-Butandiol von 1 : 350 vorlagen.

### Beispiel 3

Vergleichsbeispiel 1 wurde wiederholt, allerdings wurden zuvor alle Flansche und Stutzen (für Temperatur- und Druckmessungen) der Kolonnenkaskade mit Silikondichtmasse eingedichtet. Danach sank das molare Verhältnis von Sauerstoff zu 1,4-Butandiol im Abgas der vierten Kolonne auf ca. 1 : 1000. Das resultierende reine 1,4-Butandiol zeigte anschließend eine Reinheit von 99,8 % 1,4-Butandiol, 0,1 % 2-Methyl-1,4-butandiol, 0,05 % Acetal, 4-Hydroxybutyraldehyd bzw. dessen cyclisches Halbacetal nicht nachweisbar, 0,01 % gamma-Butyrolacton sowie mehrere weitere, mengenmäßig unbedeutende Komponenten.

## Patentansprüche

1. Integriertes Verfahren zur kontinuierlichen Herstellung von 1,4-Butandiol, das folgende Stufen umfasst,
(I) Umsetzung von Formaldehyd mit Acetylen in Gegenwart eines kupferhaltigen Katalysators bei einem pH-Wert von 5 bis 8 und einem Molverhältnis von Formaldehyd zu Acetylen von höchstens 2:1,
(II) Zwischenpufferung des erhaltenen Butindiol-haltigen wässrigen Gemischs für 0,1 bis 100h,
(III) Hydrierung des nach der Zwischenpufferung erhaltenen Gemischs und
(IV) Destillation des in Stufe III erhaltenen Hydrierprodukts zur Gewinnung von 1,4-Butandiol,
wobei der Reaktionsaustrag aus Stufe (I) direkt in die Zwischenpufferung (II) gefahren wird und nach der Zwischenpufferung (II) in mindestens einer Destillationsstufe (IIa) Acetylen, Formaldehyd, Wasser und Nebenprodukte abgetrennt werden und wobei unter Zwischenpufferung eine von dem Reaktor der Stufe (I) und den Hydrierreaktoren der Stufe (III) räumlich getrennter Bereich, der ein beliebiger Behälter, ein Tank, ein Rührapparat oder eine Rohrleitung sein kann, verstanden, in dem der Reaktionsaustrag für die erfindungsgemäße Verweilzeit verbleibt.

2. Integriertes Verfahren nach Anspruch 1, **dadurch gekennzeichnet, dass** die Zwischenpufferung bei 20 bis 100°C und einem Druck von 0,8 bis 20 bar durchgeführt wird.

3. Integriertes Verfahren nach einem der Ansprüche 1 oder 2, **dadurch gekennzeichnet, dass** die Zwischenpufferung bei 40 bis 90°C und einem Druck von 0,8 bis 20 bar durchgeführt wird.

4. Integriertes Verfahren nach einem der Ansprüche 1 bis 4, **dadurch gekennzeichnet, dass** die Zwischenpufferung bei 50 bis 80°C und einem Druck von 0,8 bis 20 bar durchgeführt wird.

5. Integriertes Verfahren nach einem der Ansprüche 1 bis 3, **dadurch gekennzeichnet, dass** die Zwischenpufferung bei einer mittleren Verweilzeit von 0,5 bis 50 h durchgeführt wird.

6. Integriertes Verfahren nach einem der Ansprüche 1 bis 5, **dadurch gekennzeichnet, dass** nach der Destillationsstufe (IIa) und vor der Hydrierung unter den Bedingungen der Ansprüche 1 bis 5 erneut zwischengepuffert wird.

7. Integriertes Verfahren nach einem der Ansprüche 1 bis 6, **dadurch gekennzeichnet, dass** über die gesamte Reaktionszone in Stufe (I) freies Acetylen vorliegt.

8. Integriertes Verfahren nach einem der Ansprüche 1 bis 7, **dadurch gekennzeichnet, dass** die Hydrierung (Stufe III) bei einem pH-Wert von 6 bis 9 durchgeführt wird.

9. Integriertes Verfahren nach einem der Ansprüche 1 bis 8, **dadurch gekennzeichnet, dass** die Hydrierung (Stufe III) in mindestens zwei Stufen durchgeführt wird.

10. Integriertes Verfahren nach einem der Ansprüche 1 bis 9, **dadurch gekennzeichnet, dass** der Katalysator der Hydrierung (Stufe III) nach Gebrauch gewaschen wird und anschließend wieder eingebaut wird.

## Claims

1. An integrated process for continuously preparing 1,4-butanediol, which comprises the following stages:
(I) reacting formaldehyde with acetylene in the presence of a copper catalyst at a pH of from 5 to 8 and a molar ratio of formaldehyde to acetylene of at most 2:1,
(II) intermediately buffering the resulting butynediol-containing aqueous mixture for from 0.1 to 100 h,
(III) hydrogenating the mixture obtained after the intermediate buffering and
(IV)distilling the hydrogenation product obtained in stage III to obtain 1,4-butanediol,
where the reaction effluent from stage (I) is conducted directly into the intermediate buffering (II) and acetylene, formaldehyde, water and byproducts are removed in at least one distillation stage (IIa) after the intermediate buffering (II) and where intermediate buffering is understood to mean a region which is spatially separate from the reactor of stage (I) and the hydrogenation reactors of stage (III) and may be any vessel, a tank, a stirred apparatus or a pipeline in which the reaction effluent remains for the inventive residence time.

2. The integrated process according to claim 1, wherein the intermediate buffering is performed at from 20 to 100°C and a pressure of from 0.8 to 20 bar.

3. The integrated process according to either of claims 1 and 2, wherein the intermediate buffering is performed at from 40 to 90°C and a pressure of from 0.8 to 20 bar.

4. The integrated process according to any of claims 1 to 4, wherein the intermediate buffering is performed at from 50 to 80°C and a pressure of from 0.8 to 20 bar.

5. The integrated process according to any of claims 1 to 3, wherein the intermediate buffering is performed at a mean residence time of from 0.5 to 50 h.

6. The integrated process according to any of claims 1 to 5, wherein there is another intermediate buffering stage under the conditions of claims 1 to 5 after the distillation stage (IIa) and before the hydrogenation.

7. The integrated process according to any of claims 1 to 6, wherein free acetylene is present through the entire reaction zone in stage (I).

8. The integrated process according to any of claims 1 to 7, wherein the hydrogenation (stage III) is performed at a pH of from 6 to 9.

9. The integrated process according to any of claims 1 to 8, wherein the hydrogenation (stage III) is performed in at least two stages.

10. The integrated process according to any of claims 1 to 9, wherein the catalyst of the hydrogenation (stage III) is washed after use and then reinstalled.

## Revendications

1. Procédé intégré pour la fabrication continue de 1,4-butanediol, qui comprend les étapes suivantes :
(I) la mise en réaction de formaldéhyde avec de l'acétylène en présence d'un catalyseur contenant du cuivre à un pH de 5 à 8 et à un rapport molaire entre le formaldéhyde et l'acétylène d'au plus 2:1,
(II) l'entreposage du mélange aqueux contenant du butynediol obtenu pendant 0,1 à 100 h,
(III) l'hydrogénation du mélange obtenu après l'entreposage, et
(IV) la distillation du produit d'hydrogénation obtenu à l'étape III pour obtenir du 1,4-butanediol,
la sortie de réaction de l'étape (I) étant introduite directement dans l'entreposage (II), et l'acétylène, le formaldéhyde, l'eau et les produits secondaires étant séparés après l'entreposage (II) en au moins une étape de distillation (IIa), et l'entreposage se rapportant à une zone séparée dans l'espace du réacteur de l'étape (I) et des réacteurs d'hydrogénation de l'étape (III), qui peut être un contenant quelconque, une cuve, un appareil agité ou une canalisation, dans laquelle la sortie de réaction demeure pendant le temps de séjour selon l'invention.

2. Procédé intégré selon la revendication 1, **caractérisé en ce que** l'entreposage est réalisé à une température de 20 à 100 °C et une pression de 0,8 à 20 bar.

3. Procédé intégré selon l'une quelconque des revendications 1 ou 2, **caractérisé en ce que** l'entreposage est réalisé à une température de 40 à 90 °C et une pression de 0,8 à 20 bar.

4. Procédé intégré selon l'une quelconque des revendications 1 à 4, **caractérisé en ce que** l'entreposage est réalisé à une température de 50 à 80 °C et une pression de 0,8 à 20 bar.

5. Procédé intégré selon l'une quelconque des revendications 1 à 3, **caractérisé en ce que** l'entreposage est réalisé pendant un temps de séjour moyen de 0,5 à 50 h.

6. Procédé intégré selon l'une quelconque des revendications 1 à 5, **caractérisé en ce qu'**un nouvel entreposage est effectué dans les conditions des revendications 1 à 5 après l'étape de distillation (IIa) et avant l'hydrogénation.

7. Procédé intégré selon l'une quelconque des revendications 1 à 6, **caractérisé en ce que** de l'acétylène libre est présent dans l'ensemble de la zone de réaction à l'étape (I).

8. Procédé intégré selon l'une quelconque des revendications 1 à 7, **caractérisé en ce que** l'hydrogénation (étape III) est réalisée à un pH de 6 à

9. Procédé intégré selon l'une quelconque des revendications 1 à 8, **caractérisé en ce que** l'hydrogénation (étape III) est réalisée en au moins deux étapes.

10. Procédé intégré selon l'une quelconque des revendications 1 à 9, **caractérisé en ce que** le catalyseur de l'hydrogénation (étape III) est lavé après l'utilisation, puis remis en place.
